# EUROPEAN PATENT APPLICATION

(11) **EP 2 524 691 A1**
(43) Date of publication of application: **21.11.2012**
(21) Application number: 11166570.9
(22) Date of filing: 18.05.2011
(51) Int. Cl.: A61K 31/05, A61K 31/695, A61K 36/82, A61K 36/87, A61K 36/81

(54) **An antioxidant composition**

(71) Applicant: Ivkovic, Slavko, 10000 Zagreb (HR)
(72) Inventor: Ivkovic, Slavko, 10000 Zagreb (HR)
(74) Representative: Thomas, Dean

(57) **Abstract**

The present invention relates to a new class of anitoxidant agent comprising silica earth, resveratrol, grape seeds extract, green tea extract and tomato powder. In particular this composition comprises between 32 to 60 percent by weight of silica earth, between 6 to 22 percent by weight of resveratrol, between 16 to 20 percent by weight of grape seeds extract, between 8 to 10 percent by weight of green tea extract, and between 10 to 16 percent by weight of tomato powder.

## Description

The present invention relates to compositions which comprise a combination of active ingredients which together have an antioxidant effect.

Radicals (often referred to as free radicals) are atoms, molecules or ions with unpaired electrons on an open shell configuration. Free radicals may have positive, negative or zero charge. Even though they have unpaired electrons, by convention, metals and their ions or complexes with unpaired electrons are not considered to be radicals. With some exceptions, the unpaired electrons cause radicals to be highly chemically reactive.

Free radicals in the body generally occur naturally as a result of metabolism. The body has built-in defences against free radicals, but these are sometimes overwhelmed by the effects of factors such as stress or environmental pollution and also their effectiveness tends to degenerate with age.

The level of free radicals in the body is also called the oxidative stress level or simply oxidative stress.

Researchers and clinicians have established that free radicals are at least one cause of several important pathologies and most notably degenerative diseases and cancer.

As a consequence of the several factors such as the majority of the world's populations increasingly urbanised existence, the amount of free radicals in the environment which people are exposed to is increasing all the time. Other reasons for this increase include the pollution of the natural environment, the degradation of the ozone layer and increases in the concentration of atmospheric CO₂. These problems are especially acute in urban areas where environmental contamination levels tend to be higher.

In addition to such exogenous sources of free radicals a population of free radicals are also constantly produced endogenously, which also have an influence on oxidative stress. In particular the majority of free radical species arise during the course of respiration and its associated processes in each cells mitochondria. The reactive oxygen intermediates O₂-, OH-, NO- and other "non-radical" oxygen derivatives such as hydrogen peroxide, H₂O₂ and hypochlorous acid (HClO) are all produced and participate in reactions that lead to increased levels of endogenous free radicals.

Therefore cells and tissues are constantly exposed to both exogenous and endogenous oxygen radicals and as a consequence are in constant danger from oxidative damage.

A number of biological systems exist such as the enzymes SOD, GSH-Px and metaloprotein ceruloplasmin. In addition a number of substances are also known to have antioxidant properties such as Vitamin E, Vitamin C, carotenoids, flavonoids, uric acid, bilirubin, glutathione.

Observing the increasing problems of high levels of oxidative stress, the inventor has set out to develop a composition which can reduce oxidative stress levels and therefore the inventors have now developed a new composition which shows high levels of antioxidative activity.

According to a first aspect of the present invention there is provided an anitoxidant agent comprising of silica earth, resveratrol, grape seeds extract, green tea extract and tomato powder.

In particular there is provided an antioxidant agent consisting of silica earth, resveratrol, grape seeds extract, green tea extract and tomato powder

In particular this composition comprises:
between 32 to 60 percent by weight of silica earth,
between 6 to 22 percent by weight of resveratrol,
between 16 to 20 percent by weight of grape seeds extract,
between 8 to 10 percent by weight of green tea extract, and
between 10 to 16 percent by weight of tomato powder.

In accordance with the present invention each of the components of the composition are defined as follows.

Silica earth also sometimes called Amorphous or Diatomaceous Earth is a soft siliceous solid composed of the skeletons of small prehistoric aquatic plants and it contains primarily silica.

Silca earth has been used as an aid in the clearance of digestive parasites in animals for many years and it is also used as an aid to human digestion and silica earth is therefore safe for human consumption.

Silica earth comprises complex microscopic structures which present an enormous surface area (up to 1,000 m²/g) and also have cation bonding capacity. Silica earth can therefore bind to and so eliminate free radicals. Although the area of effect for the silica earth is largely limited to the gastrointestinal tract (GI), even in this limited area of the body it can significantly reduce the resulting damage to biological structures by free radicals such lipid peroxidation of intestinal cell membranes and oxidation of genomic DNA. The damage caused by free radicals in the GI are not limited to the GI, due to the absorption via the intestinal wall of peroxidised fatty acids, a product of the reaction between free radicals and lipids such as those in the cell membranes of the cells lining the GI.

Resveratrol (3,5,4'-trihydroxy-trans-stilbene) is a stilbenoid, a type of natural phenol and a phytoalexin produced naturally by several plants when under attack by pathogens such as bacteria or fungi.

Resveratrol is currently the topic of numerous animal and human studies into its effects and in particular the effects of resveratrol upon the lifespan of many model organisms as well as anti-cancer, anti-inflammatory, blood-sugar-lowering and other beneficial effects particularly on the cardiovascular system have been reported in several models, but these results have yet to be replicated in humans.

Resveratrol is found in the skin of red grapes and in other fruits such as Japanese knotweed. Red wine contains a high level of it and some scientists believe it is one of the factors behind the French Paradox, that is lower than expected levels of heart disease, diabetes and obesity even though the French diet is relatively rich in saturated fats. Resveratrol has also been produced by chemical synthesis and obtained from engineered microorganisms.

In accordance with a further aspect of the present invention the tomato powder comprises lycopene.

Lycopene is a bright red carotene and carotenoid pigment and phytochemical found in tomatoes and other red fruits and vegetables, such as red carrots, watermelons and papayas but not strawberries or cherries. Although lycopene is chemically a carotene, it has no vitamin A activity.

Lycopene is not an essential nutrient for humans, but is commonly found in the diet, mainly from dishes prepared with tomato sauce. When absorbed from the stomach, lycopene is transported in the blood by various lipoproteins and accumulates in the liver, adrenal glands, and testes.

Workers noted in previous research an inverse relationship between the consumption of tomatoes and the risk of developing cancer, following on from this severak groups have investigated lycopene as a potential agent for prevention of some types of cancers, particularly prostate cancer. However, the effects of lycopene upon the development of prostate cancer was deemed insufficient evidence for the approval health claim by the US Food and Drug Administration.

Grape seed extracts particularly flavonoids and even more specifically oligomeric procyanidins (OPCs), are a class of flavanols that consist essentially of polymer chains of flavonoids.

OPCs like resveratrol are found in the skin of red grapes and in other fruits and OPCs are another component of red wine which some scientists believe to be one of the factors behind the French Paradox. In addition OPCs are known to be able to prevent and repair capillary damage.

Green tea is tea made solely with the leaves of *Camellia sinensis* that have undergone minimal oxidation during processing.

Over the last few decades green tea has been subjected to many scientific and medical studies to determine the extent of its long-purported health benefits, with some evidence suggesting that regular green tea drinkers have lower chances of heart disease and developing certain types of cancer.

Green tea extracts include green tea polyphenols, such as epigallocatechin gallate (EGCG), epigallocatechin (EGC), epicatechin gallate (ECG) and epicatechin (EC).

In accordance with a further aspect of the present invention the grape seed extract comprises flavonids and preferably oligomeric procyanidins (OPCs).

In accordance with a further aspect of the present invention the green tea extract comprises green tea polyphenols, and preferably at least one green tea polyphenol selected from the group epigallocatechin gallate (EGCG), epigallocatechin (EGC), epicatechin gallate (ECG) and epicatechin (EC).

The composition may be formulated so as to aid the molecular mechanisms that maintain healthy cells from free radicals and particularly oxidative damage, in particular the formulation is intended to prevent the formation of mutated/oncogenic cells, neurodegenerative diseases and autoimmune diseases and thus leads to a prolonged healthy life.

The composition according to the present invention represents a new class of antioxidant that stimulates cellular antioxidant enzymes such as:
- Superoxide Dismutase (SOD) 1, dysfunction of SOD1 has been associated with a number of diseases including Amyotrophic Lateral Sclerosis a form of motor neuron disease caused by the degeneration of neurons located in the ventral horn of the spinal cord and the cortical neurons that provide their afferent input.
- SOD2, dysfunction of SOD2 has been associated with idiopathic cardiomyopathy, sporadic motor neuron disease, and cancer. Studies have also hown that mice which are 50% deficient in SOD2 suffer increased DNA damage and increased incidence of cancer.
- SOD3, SOD3 is thought to protect the brain, lungs and other tissues from oxidative stress and SOD3 dysfunction has been linked to ischemic heart disease.
- Glutathione peroxidase (GPX) are an enzyme family with peroxidase activity whose main biological role is to protect the organism from oxidative damage. The biochemical function of glutathione peroxidase is to reduce lipid hydroperoxides to their corresponding alcohols and to reduce free hydrogen peroxide to water. Dysfunction of member of the GPX family has been linked to damage to brain cells and idiopathic anemia.
- Glutathione reductase (GSR) dysfunction of GSR has been linked to declines in renal function and the occurrence of Nephroblastoma of the kidneys of children.

In addition the Inventor also considers their composition to have several further advantages such as the ability to:
- Slow down the aging process by protecting the molecular mechanisms that maintain the cells ability to regenerate by mitosis as well as to grow and differentiate,
- Protect the molecular mechanisms of programmed cells death (p21, p27, p53 and JNK1 gene),
- Protect the molecular mechanisms of DNA, RNA and proteins damage repair (enhance Trans-Lesion Synthesis),
- Prevent the production of mutated cells (reduce the incidence of cancer),
- Prevent post inflammtory oxidative damage and chronic changes that would produce chronic changes in liver, kidney, heart, joints.

The inventors also consider the composition according to the present invention to be useful in the treatment/alleviation of symptoms of a number of other diseases such as neuronal diseases like Schizophrenia, Alzheimer's disease, Parkinson's disease, depression, neuronal damage, multiple sclerosis, epilepsy, dementia, muscular dystrophy, idiopathic cardiomyopathy, mitochondrial myopathy, Fridreich myopathy, coronary and cerebral arteriosclerosis. As well as cancer and proliferative diseases such as melanoma, lung carcinoma, kidney cancer, breast cancer and prostate cancer. As well as diseases of the eye such as cataract, muscular degeneration, diabetic retinopathy, ocular hemography and diseases of the heart, cardio vascular system and respiratory diseases such as heart disease and respiratory cystic fibrosis, adult respiratory distress syndrome-asthma, lung cancer, emphysema, hyperoxia, bronchopulmonary dysplasia, Atherosclerosis, myocardial infarction, ischemia, cardiomyopathy, hypertension and thrombosis. The composition is also suitable for treating/alleviating the symptoms of diseases of the liver such as viral hepatitis, liver cirrhosis and autoimmune diseases such as rheumatoid arthritis, burning mouth syndrome, lupus erythematosus, skin diseases such as eczema, melanoma, porphyrias, contact dermatitis and sterility.

Some animal studies have suggested that reactive radicals contribute to the destruction of pancreatic islet cell in the pathogenesis of insulin dependent diabetes (type 1). Therefore the composition according to the present invention is also considered suitable for treating and/or alleviating the symptoms of type 1 diabetes.

According to a further aspect of the present invention the composition according to the present invention may also comprise additional auxiliary materials which are well known in the pharmaceutical art such as carriers, adjuvants and/or vehicles.

The carriers can be for example, fillers, extenders, binders, humectants, disintegrating agents, solution retarders, absorption enhancers, wetting agents, adsorbents and/or lubricants.

In accordance with a further aspect of the present invention there is provided a pharmaceutical composition comprising the antioxidant composition according to the first aspect of the present invention.

The pharmaceutical composition is provided as a gel, powder, tablet, sustained-release tablet, premix, emulsion, infusion formulation, drops, concentrate, granulate, syrup, pellet, boluses, capsules, aerosol, spray and/or inhalant for use in this form or as a constituent of a further formulation intended for use.

The tablets, pills, capsules and granules according to the present invention can further comprise coatings and internal shell structures so that the active ingredient is only or is preferably released in a particular part of the intestinal tract. Alternatively the tablets, pills, capsules and granules according to the present invention can also comprise appropriate means so as to delay the release of the active ingredient and/or embedding materials such as organic polymeric substances like wax.

In particular therefore the present invention relates to a product, for oral administration in any orally acceptable dosage form, including capsules, tablets and aqueous suspensions and solutions, but not limited to any of these forms.

In the case of tablets for oral use, carriers that are used frequently include lactose and corn starch.

Capsules may also comprise diluents such as lactose and dried corn starch, lubricants such as magnesium stearate, can also be added to tablets or capsules.

For aqueous suspensions to be administered orally, the composition may be mixed with emulsifying and suspending agents.

If desired, certain sweetening and/or flavoring and/or coloring agents may also be added to any of these specific formulations.

The composition can be present with one or more of the above mentioned carrier substances in a micro-encapsulated form.

According to another aspect of the present invention the composition can be in the form of suppositories. Suppositories can contain the composition and conventional water-soluble or water-carriers such as polyethylene glycols, fats, such as cocoa fat and higher esters (for example C14-alcohol with C16 fatty acids or mixtures of these substances).

Ointments, pastes, creams and gels, in addition to or contain the composition may also comprise excipients, such as animal and vegetable fats, waxes, paraffins, starch, tragacanth, cellulose derivatives, polyethylene glycols, silicones, bentonites, silicic acid, talc and zinc oxide or mixtures of these substances.

Powders and sprays can contain the composition in addition to carriers, such as lactose, talc, silica, aluminum hydroxide, calcium silicate and Polyamide powder or mixtures of these substances. Sprays can additionally contain the propellants, such as chloro-fluorocarbons.

Solutions and emulsions, in addition to the composition, may also comprise, conventional carriers such as solvents, solubilizing agents and emulsifiers email, for example water, ethyl alcohol, isopropyl alcohol, ethyl carbonate, ethyl acetate, Benzyl alcohol, benzyl benzoate, propylene glycol, 1,3-butylene glycol, dimethylformamide, oils, particularly cottonseed oil, peanut oil, corn oil, olive oil, castor oil and sesame oil, glycerol, glycerol formal containing tetrahydrofurfuryl alcohol, polyethylene glycols and fatty acid esters of sorbitan or mixtures of these substances. For parenteral administration, the solutions and emulsions in sterile and blood-isotonic form.

Suspensions, in addition to the composition include conventional carriers such as liquid diluents, such as water, ethyl alcohol, propylene glycol, suspending agents, ethoxylated isostearyl alcohols, polyoxyethylene sorbitol and sorbitan esters, microcrystalline cellulose, bentonite, agar-agar and tragacanth or mixtures of these substances.

According to another aspect of the present invention the pharmaceutical composition can be in the form of an injectable drug such as a lyophilized sterile injectable formulation, as a sterile injectable aqueous or oily suspension. This suspension may also be made using suitable dispersing or wetting agents such as Tween 80 and suspending agents. The sterile injectable preparation may also be a sterile injectable solution or suspension in a nontoxic parenterally acceptable diluent or solvent, for example a solution in 1,3-butanediol. Among the acceptable vehicles and solvents that may be employed are mannitol, water, Ringer's solution and isotonic sodium chloride solution. Furthermore, sterile, fixed oils are used as a solvent or suspending medium. For this purpose any non-volatile oil, including synthetic mono-or diglycerides may be used. Fatty acids such as oleic acid and its glyceride derivatives are useful in the preparation of injectables, as are natural pharmaceutically acceptable oils such as olive oil or castor oil, especially in their polyoxyethylated forms. These oil solutions or suspensions may also contain a long chain alcohol or a similar alcohol as a diluent or dispersant.

The formulations mentioned above can also include colorants, preservatives, odor or taste compounds, for example peppermint oil and eucalyptus oil and sweeteners, such as saccharin.

The pharmaceutical composition according to the present invention may comprise the composition (that is the combination of the listed agents) at a concentration of between 0.01 to 99.9 by final weight and preferably from between 0.05 to 99 by final weight.

The composition may also contain other active pharmaceutical ingredients, such as pharmaceutical agents and there salts, buffers, vitamins, sugars and there derivatives, especially saccharides, enzymes, plant extracts.

The pharmaceutical composition and specific formulations according to the present invention can in humans or other animals be administered either orally, rectally, parenterally (intravenous, intramuscular, subcutaneous), intracistemal, intravaginally, intraperitoneally, locally (powders, ointments, drops) and are used for the treatment of the diseases mentioned above.

As suitable preparations for injection solutions, solutions and suspensions for oral therapy, gels, infusion formulations, emulsions, ointments or drops. For local therapy, ophthalmic and dermatological formulations, silver and other salts, ear drops, eye ointments, powders or solutions can be used. In animals, the composition may also be administered via the animal feed or drinking water in suitable formulations. Furthermore, the composition may be incorporated into other materials such as plastics/biomaterials which can in turn be used for reconstructive surgery such as collagen or bone/joint replacements.

In another preferred embodiment of the invention are the pharmaceutical composition is in a concentration of 0.1 to 99.5 by weight and preferably from 0.5 to 95 by weight and most preferably 20 to 80 by weight in a pharmaceutical preparation.

The composition can be used in accordance with a preferred embodiment, in a total amount of 0.05 to 500 mg / kg body weight every 24 hours, preferably from 5 to 100 mg / kg body weight.

For a better understanding of the invention and to show how the same may be carried into effect, there will now be shown by way of example only, specific embodiments, methods and processes according to the present invention with reference to the accompanying drawings in which:
Fig. 1 Total antioxidant status measurement of HPE 40
Fig. 2 Estimation of the trolox activity for the HPE 30 with the 2^{nd} order polynomial
Fig. 3 Estimation of the trolox activity for the HPE 40 with the 2^{nd} order polynomial
Fig. 4 The inhibition of poly-L-arginine carbonylation *in vitro,* wherein the level of carbonylation is determined by absorbance at 450 nm
Fig 5. The inhibition of *E.coli* carbonylation *in vitro* wherein the level of carbonylation is determined by absorbance at 450 nm
Fig. 6 The inhibition of alpha-synuclein carbonylation *in vitro* wherein the level of carbonylation is determined by absorbance at 450 nm
Fig. 7 The inhibition of tau protein carbonylation *in vitro,* wherein the level of carbonylation is determined by absorbance at 450 nm
Fig. 8 The inhibition of HeLa cells carbonylation *in vitro,* wherein the level of carbonylation is determined by absorbance at 450 nm
Fig. 9 Comparison of antioxidant capacity of HPE 40 and commercially available multivitamin compositions by measuring the inhibition of poly-L-arginine carbonylation *in vitro* wherein the level of carbonylation is determined by absorbance at 450 nm
Fig. 10 Comparison of antioxidant capacity of HPE 40 and commercially available multivitamin compositions by measuring the inhibition of *E.coli* carbonylation *in vitro* wherein the level of carbonylation is determined by absorbance at 450 nm
Fig. 11 Comparison of Total antioxidant capacity of HPE 40 and commercially available multivitamin compositions by measurement of trolox activity.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### EXAMPLE 1. - EFFECTS OF ANTIOXIDANT AGENT ON OXYGEN FREE RADICALS

### 1.1 RESULTS

The tested samples were evaluated for their total antioxidant status as well as their potential to inhibit protein carbonylation *in vitro.*

In order to measure total antioxidant status of the samples a Randox TAS kit was used. Shortly, the main principle of the method is the following: ABTS® (2,2'-Azino-di-[3-ethylbenzthiazoline sulphonate]) is incubated with a peroxidase (metmyoglobin) and H₂O₂ to produce the radical cation ABTS®*+. This has a relatively stable blue-green color, which is measured at 600 nm. Antioxidants in the added sample cause suppression of this color production to a degree, which is proportional to their concentration.

The level of protein carbonylation was measured using the OxyELISA kit available at Millipore (S7250). The potential of the samples to inhibit protein carbonylation was tested in two systems:
i) *in vitro* oxidation of poly-L-arginine, in which all amino acids are a target for carbonylation, and
ii) protein extract of exponentially growing *Escherichia coli.*

Exponentially growing bacteria were harvested from a rich medium (LB for), centrifuged and re-suspended in the lysis buffer available in the OxyELISA kit supplemented with a mixture of protease inhibitors (Roche). Cells were broken by two freeze-thaw cycles, homogenized in a Dounce homogenizer, and centrifuged 20 min at 12,000 × g. The amount of protein in the supernatant was measured by the Lowry method (Lowry OH, et al., 1951, Protein measurement with the Folin phenol reagent. JBiol Chem 193:265-275*)* using the OxyElisa kit.

Both, poly-L-arginine and the *E.coli* protein extracts were diluted to 10 µg/mL and loaded into wells (provided in the kit) and incubated over night at 4 °C to allow proteins to adsorb to the surface, followed by DHR derivatization of adsorbed proteins and detection of derivatized dinitrophenol (DNP)-carbonyl by a mouse DNP-specific monoclonal antibody conjugated to HRP. Subsequent incubation with enzyme substrate 3,3',5,5'-tetramethylbenzidine resulted in a colored product that was quantified using a micro plate reader with maximum absorbance at 450 nm. Protein samples were oxidized by using hydrogen peroxide in the final concentration of 100 µM during 15 minutes at room temperature. The reaction was stopped by cooling the mixture to 4°C.

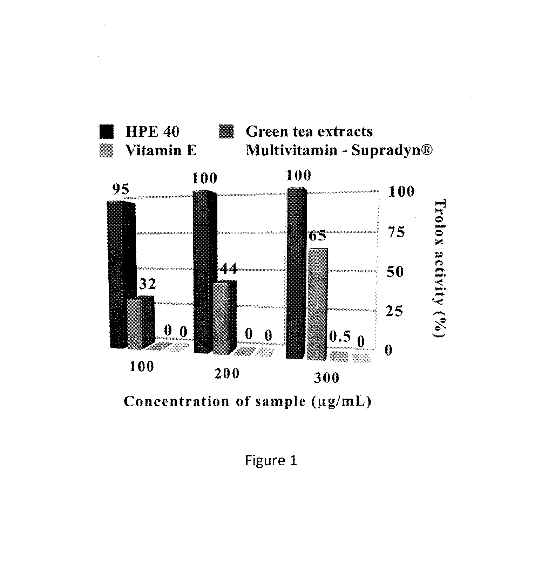

The achieved results show that the HPE 40 is a highly potent antioxidant. The composition in the concentration of 300 µg/mL (equivalent of 3 capsules per day) reaches up to 100 % of the control (Fig. 1).

The HPE 40 composition used in these experiments comprised:
- 51 percent by weight of silica earth,
- 7 percent by weight of resveratrol,
- 20 percent by weight of grape seeds extract,
- 10 percent by weight of green tea extract, and
- 12 percent by weight of tomato powder.

It should be noted that the control is a solution of trolox (a water soluble analogue of vitamin E) and this is defined as 100% antioxidant capacity. Interestingly, the concentration of trolox usually used in the kit is approximately 180 times higher than the recommended daily dose of vitamin E. Therefore, for comparison, we have looked at the antioxidant capacity of a 180-fold dilution of the control. In addition, for comparison, we have presented the results for a commercially available green tea extract, vitamin E and a multivitamin tablet. The concentrations tested are 100 µg/mL, 200 µg/mL and 300 µg/mL, the equivalent of 1, 2 and 3 500 mg capsules per day, respectively.

As it can be seen from Fig. 1 HPE 40 in the concentration of 200 µg/mL reaches 100% of the control, i.e. a solution of trolox. It is obvious that this method cannot accurately estimate the percentage of the trolox activity of the HPE 40. That is why the trolox activity has been estimated on the basis of the analysed trolox activity of another next-generation antioxidant (HPE 30).

The HPE 30 composition used in these experiments comprised:
- 51 percent by weight of silica earth,
- 5 percent by weight of L-arginin,
- 2.5 percent by weight of L-glutamin,
- 3.5 percent by weight of resveratrol,
- 19.5 percent by weight of grape seeds extract,
- 5 percent by weight of lemon balm, and
- 13.5 percent by weight of tomato powder.

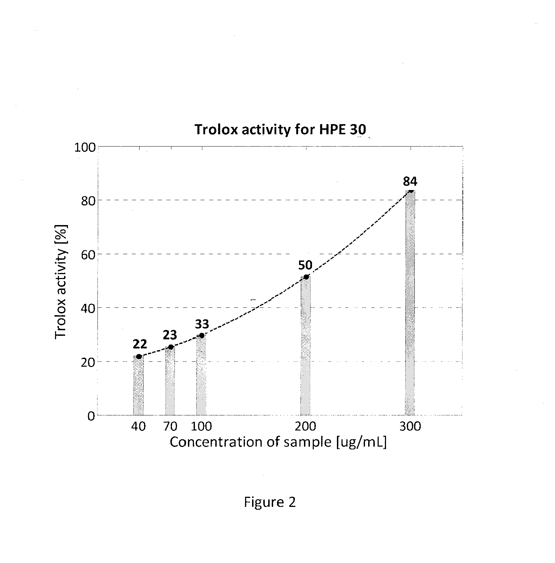

The measured results for this antioxidant were approximated using a second order polynomial function (Fig. 2), which has showed good data fitting (confirmed by the low Normalised Root Mean Square factor, NRMS=0.082).

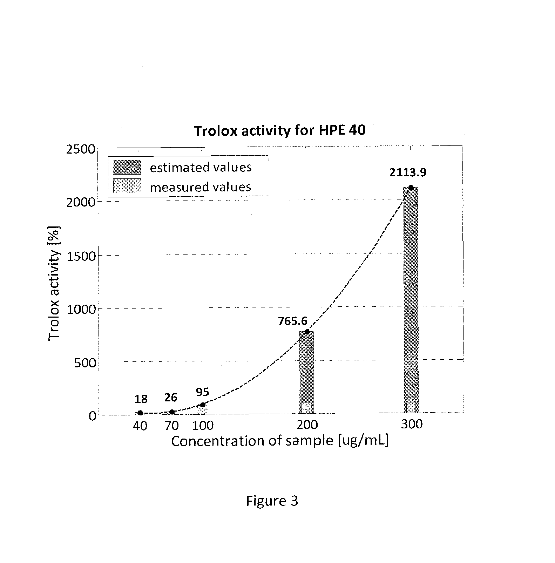

Using the same order of the polynomial and the measured data for the 40, 70 and 100 µg/mL sample concentrations, trolox activity for the concentrations of 200 and 300 µg/mL are estimated, these estimated values are presented in Fig. 3. These results show extraordinary high antioxidative effect - daily dosage of the HPE 40 is approximately 3800 higher than the daily dosage of vitamin E (2113.9% of the trolox activity).

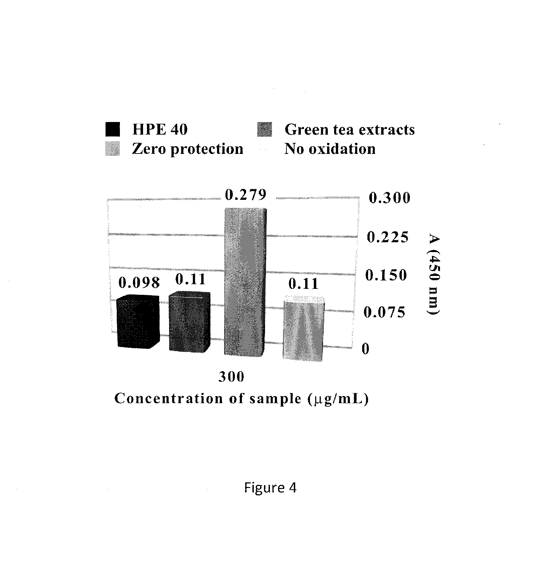

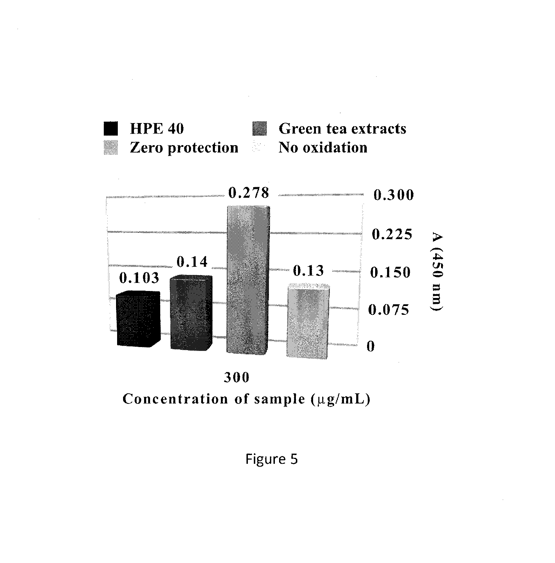

HPE 40 sample was also the most potent sample to inhibit protein carbonylation *in vitro* (Figs. 4, 5). It should be noted that two very important controls are included in this experiment. One is a positive control wherein no sample was added, that is, a control for maximum oxidation and no protection. In this sample maximum carbonylation is achieved under the condition applied in these experiments. In addition, there is a negative control for zero oxidation where no hydrogen peroxide was added. This control serves as a minimum oxidation control under the condition applied in these experiments.

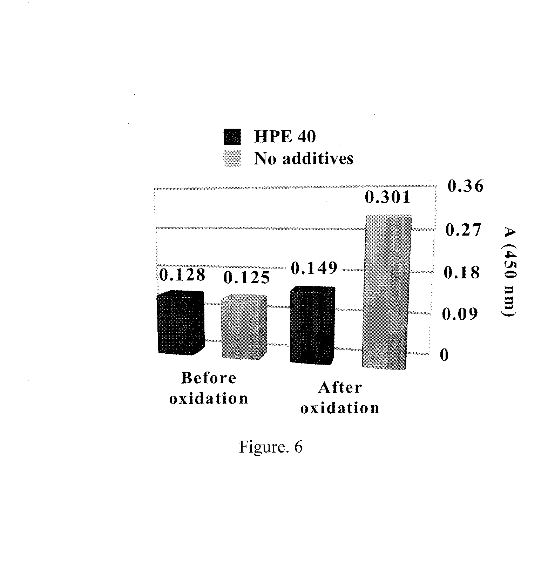

Also, the carbonylation test of the alpha-synuclein protein (Parkinskon disease) was performed. The protein (10 µg/mL) was oxidated with 100 µM hydrogen peroxide within 20 minutes at the room temerature. The results (Fig. 6) show high capability of HPE 40 (300 µg/mL) in the protection of alpha-synuclein protein from the carbonilation, which was expected regarding the high estimated percentage of trolox activity (Fig. 3).

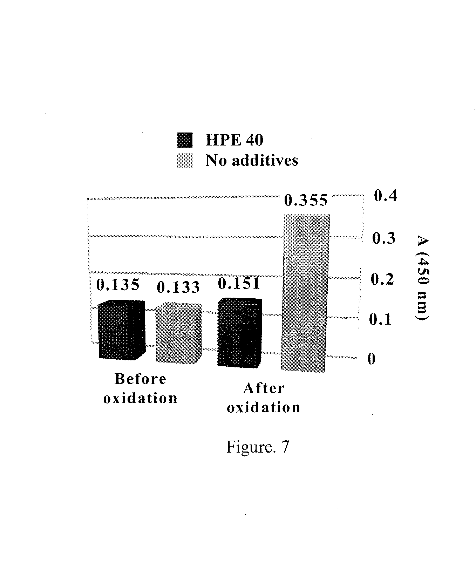

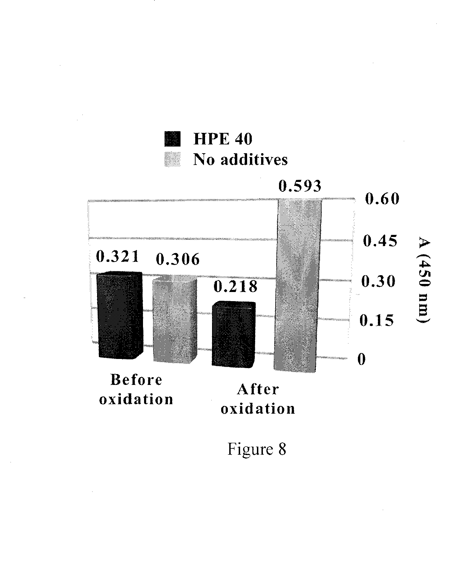

Several carbonilation tests of alpha-synuclein protein (Parkinskon disease), tau protein (Alzheimer disease) and HeLa cells were performed. Alpha-synuclein protein (10 µg/mL) was oxidated with 100 µM hydrogen peroxide within 20 minutes at the room temperature, while tau protein and HeLa cells within 1 hour. The results (Fig. 6-8) show high protective capability of HPE 40 (300 ug/mL) from the carbonilation process, which was expected regarding the high estimated percentage of trolox activity (Fig. 3). Only 25% of HeLa cells survived in the case of no protection, while 90% of them survived when they were protected with HPE 40.

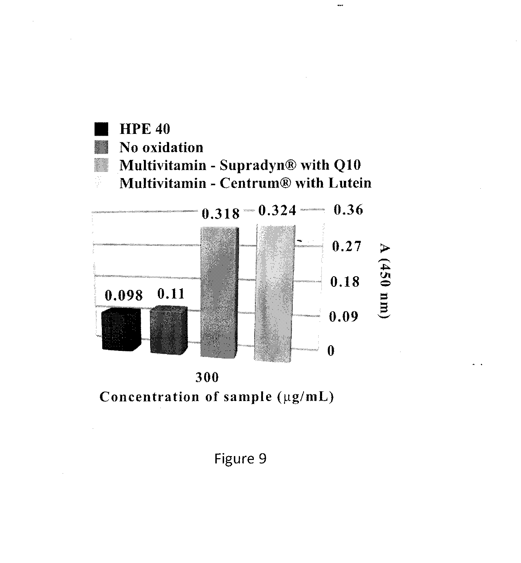

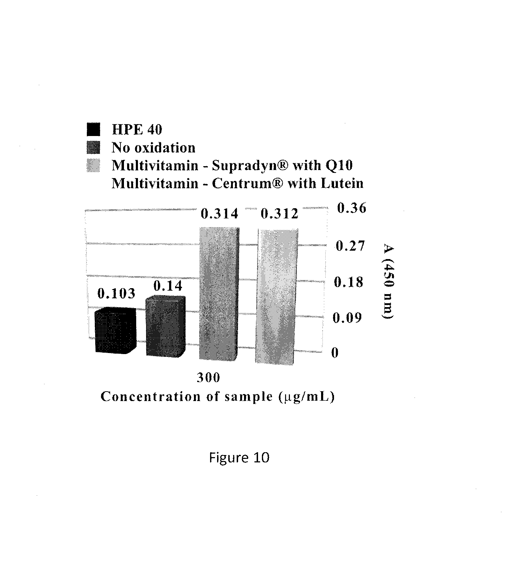

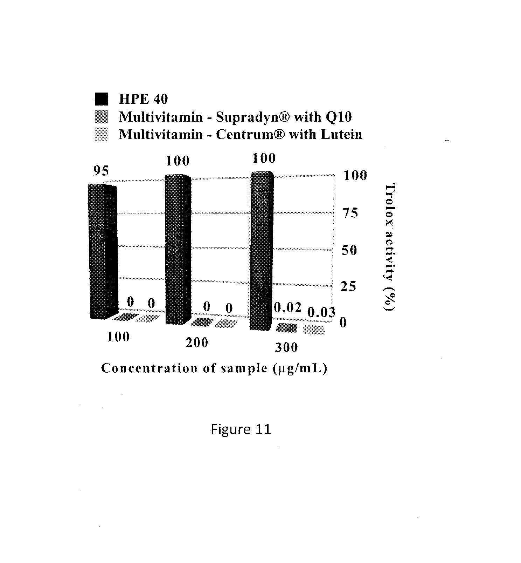

Additional tests were performed in order to compare non-vitamin antioxidant composition HPE 40 with 2 multivitamin compositions. The results are presented in Fig. 9-11. The compositions were also compared using the results of tests for inhibition of poly-L-arginine and *E.coli* proteins carbonylation, and total antioxidant status measurements. The tests were prepared and performed using the same previously explained experiment characteristics (protocol) upon which the results presented in Figures 1, 4 and 5 are obtained. All three tests have shown that both type of multivitamin dietary supplements, Centrum® with Lutein and Supradyn® with Q10, which are composed from a number of today recognised antioxidant constituents (Vitamin E, Vitamin C, Q10, Lutein, ...) have shown inferior antioxidative activity compared to HPE 40. They did not protect poly-L-arginine and *E.coli* proteins from the oxidation - carbonylation process. Also, in the total antioxidative status measurement test, they show practically no antioxidative activity even at the highest concentration of sample (0.02% and 0.03% of trolox activity).

### 1.2 ANALYSIS

The results of these experiments confirm that:
1) The best known and recognized antioxidants (vitamins A, C, E and coenzyme Q10) have minimal much lower antioxidative effect probably meaning they have no therapeutic significance.
2) All conducted clinical studies related to the prevention of diseases with these known antioxidants have so far shown negative results.
3) The new class of antioxidant developed by the applicants shows vastly increased levels of antioxidative and also anti-protein carbonylation performance in comparison to all these known products.
4) This new class of product therefore provides for the first time a meaningful way in which the effects of oxidative stress can be controlled.

This antioxidant agent (HPE 40) has a strong influence on cell activity and vitality. It is produced using the technological process which enables the high synergistic effect of the combination of the used antioxodative Active Pharmacological Ingredients (APIs). The efficiacy of this composition is significantly increased compared with the same combination of substances whose action is based solely on the additive effect of each used API.

It can be concluded that the HPE 40 agent represents the next-generation of antioxidants has the strongest known antioxidative and antiaging efficacy, showing an approximately 3800 times stronger effect than vitamin E. It protects proteins from the carbonylation process which is the main cause of the damages of intracellular molecular structures.

### EXAMPLE 2. COMPARISON OF THE LEVELS OF ANTIOXIDANT ACTIVITY OF HPE 40 AND ITS CONSTITUENTS

### 2.1 RESULTS

The total antioxidant status (refered to as TAS hereafter), measured using Randox TAS kit (see example 1), of each constituent of the HPE 40 composition and HPE 40 itself are presented in Table 1 for different concentrations of samples. The antioxidant activity is defined in the form of the fraction of trolox activity expressed as a percentage.

**Table 1 Total antioxidant activity of HPE 40 and its ingredients**

| **Total Antioxidant Status (TAS), fraction of trolox activity (%)** | | | |
|---|---|---|---|
| Sample | Concentrations (µg/mL) | | |
| | 100 | Concentrations 200 | 300 |
| HPE 40 | 95 | APprox. 765.5 (100-measured) | Approx. 2113.9 (100- measured) |
| Silica earth | 0 | 0 | ~0 |
| Resveratrol | - | - | 30 |
| Grape seeds | 0 | 0 | 17 |
| Green tea extract | 32 | 44 | 65 |
| Tomato powder | - | - | 25 |

These results show higher antioxidant activity of HPE 40 than the antioxidant activity of each constituent separately. If the results of all constituents for the concentration 300 µg/mL are added, then the total sum of trolox activity is 137% (0% + 30% + 17% + 65% + 25%), which is much smaller than the 2113.9% (approximated value) of trolox activity obtained with the HPE 40.

Even if the result of trolox activity for green tea extract, silica earth and grape seeds at the concentration of 100 µg/mL is added to the best results of resveratrol and tomato powder obtained at the highest concentrations (300 µg/mL), the sum of trolox activity (0% + 30% + 0% + 32% + 25% = 87%) is still significantly lower than the one measured for the HPE 40 at the concentration of 100 µg/mL which is 95%.

It is also important to notice that in all these comparisons the concentration of each individual constituent (100, 200 or 300 µg/mL) are several times higher than their actual concentration when present in the composition. For example, when comparing 300 µg/mL of the resveratrol with the same concentration of HPE 40, there is only 21 µg/mL (7%) of the resveratrol in the tested HPE 40 composition.

### 2.2 ANALYSIS

It is clear therefore that the HPE 40 composition has higher than expected antioxidant effects in comparison to its constituents. This enhanced antioxidant activity is caused by the synergistic effect of all constituents, in particular the inventor considers this synergistic effect to result from the use of silica earth as the main component of HPE 40. Silica earth in the combination with aforementioned non-vitamin antioxidant constituents has not yet been used in the creation of compounds with such high antioxidative effect.

## Claims

1. An antioxidant agent **characterised in that** it comprises resveratrol, grape seeds extract, green tea extract, tomato powder and silica earth.

2. The antioxidant agent of claim 1, consisting of:
between 32 to 60 percent by weight of silica earth,
between 6 to 22 percent by weight of resveratrol,
between 16 to 20 percent by weight of grape seeds extract,
between 8 to 10 percent by weight of green tea extract, and
between 10 to 16 percent by weight of tomato powder.

3. A pharmaceutical composition **characterised in that** it comprises the antioxidant agent according to claim 1 or 2.

4. The pharmaceutical composition according to claim 3, **characterized in that** it is in a form selected from the group consisting of: a gel, a powder, infusion, a tablet, a sustained-release tablet, a pre-mix, an emulsion, a drop, a concentrate, a granule, a syrup, a pellet, a bolus, a capsule, an aerosol, a spray and / or an inhalant.

5. The pharmaceutical composition according to claim 3 or 4, wherein it is in a form suitable to be administered orally, subcutaneously, intravenously, intramuscularly, intraperitonealy and/or intradermaly.

6. The pharamaceutical composition according to any one of claims 3 to 5 wherein said composition comprises between 500 - 1500 mg of said antioxidant agent according to claims 1 or 2.

7. The antioxidant agent according to claim 1 or 2 for use as a medicament.

8. The antioxidant agent according to claim 1 or 2 for curing or preventing a disease, pathology or condition selected from the group consisting of: Schizophrenia, Alzheimer's diease, Parkinson's disease, depression, neuronal damage, multiple sclerosis, epilepsy, dementia, muscular dystrophy, Amyotrophic Lateral Sclerosis (ALS), Idiopathic cardiomyopathy, Mitochondrial myopathy, Fridreich myopathy), Coronary and cerebral arteriosclerosis, melanoma metastaticum, lung carcinoma, lung cancer (microcellular and planocellular), kidney cancer (adenocarcinom), breast cancer and prostate cancer,cataracts, muscular degeneration, diabetic retinopathy, ocular hemography, heart disease and respiratory cystic fibrosis, adult respiratory distress syndrome-asthma, lung cancer, emphysema, hyperoxia, bronchopulmonary dysplasia, Atherosclerosis, myocardial infarction, ischemia, cardiomyopathy, hypertension and thrombosis,viral hepatitis, liver cirrhosis, rheumatoid arthritis, burning mouth syndrome, Lupus erythematosus, diabetes, skin diseases (eczema, melanoma, porphyrias, contact dermatitis), sterility.
